(19) Europäisches Patentamt
European Patent Office
Office européen des brevets

(11) Veröffentlichungsnummer: 0 364 731 A1

# (12) EUROPÄISCHE PATENTANMELDUNG

(21) Anmeldenummer: 89116863.5

(22) Anmeldetag: 12.09.89

(51) Int. Cl.5: C07C 217/10 , C07D 295/12 , A61K 7/13

(30) Priorität: 21.09.88 DE 3831977

(43) Veröffentlichungstag der Anmeldung: 25.04.90 Patentblatt 90/17

(84) Benannte Vertragsstaaten: ES GR

(71) Anmelder: Henkel Kommanditgesellschaft auf Aktien
Henkelstrasse 67
D-4000 Düsseldorf 13(DE)

(72) Erfinder: Rose, David, Dr.
Am Eichelkamp 223
D-4010 Hilden(DE)
Erfinder: Höffkes, Horst, Dr.
Carlo-Schmid-Strasse 113
D-4000 Düsseldorf(DE)
Erfinder: Lieske, Edgar
Hunsrückenstrasse 40
D-4000 Düsseldorf(DE)

## (54) 2-(Alkoxyalkylaminomethyl)-4-aminophenole und deren Verwendung in Oxidationshaarfärbemitteln.

(57) 2-(Alkoxyalkylaminomethyl)-4-aminophenole der Formel

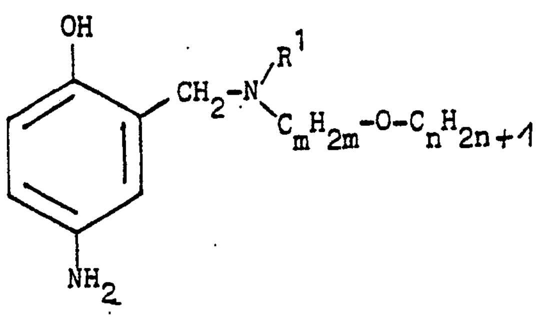

in der $R^1$ Wasserstoff, eine Alkylgruppe mit 1 - 4 C-Atomen, eine Hydroxyalkylgruppe mit 2 - 4 C-Atomen, eine Aminoalkylgruppe mit 2 - 4 C-Atomen, die am Stickstoff durch eine oder zwei Alkylgruppen mit 1 - 4 C-Atomen substituiert sein kann oder deren Stickstoffatom einen Piperidin-, Morpholin-, Pyrrolidin- oder Piperazin-Ring angehört oder eine Gruppe $-C_mH_{2m}-O-C_nH_{2n+1}$ ist, worin m eine ganze Zahl von 2 - 4 und n eine ganze Zahl von 1 - 4 ist und deren wasserlösliche Salze sind Entwickler für Oxidationsfarbstoffe und eignen sich als Oxidationsfarbstoffverprodukte zur Herstellung von Haarfärbemitteln. Sie bilden mit üblichen Kupplerverbindungen braune bis rote Nuancen mit guten Echtheitseigenschaften.

EP 0 364 731 A1

# 2-(Alkoxyalkylaminomethyl)-4-aminophenole und deren Verwendung in Oxidationshaarfärbemitteln

Gegenstand der Erfindung sind neue 2-(Alkoxyalkylaminomethyl)-4-aminophenole und deren Salze sowie deren Verwendung als Entwicklerkomponente in Oxidationshaarfärbemitteln.

Für das Färben von Haaren spielen die sogenannten Oxidationshaarfärbemittel wegen ihrer intensiven Farben und guten Echtheitseigenschaften eine bevorzugte Rolle. Solche Haarfärbemittel enthalten Oxidationsfarbstoffvorprodukte in einem kosmetischen Träger. Als Oxidationsfarbstoffvorprodkte werden Enwicklersubstanen und Kupplersubstanzen eingesetzt. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf menschlichem Haar besitzen, wobei keine merklichen Unterschiede zwischen strapaziertem und frisch nachgewachsenem Haar bestehen dürfen. Sie sollen beständig sein gegen Licht, Wärme und den Einfluß chemischer Reduktionsmittel, z.B. gegen Dauerwellflüssigkeiten. Schließlich sollen sie die Kopfhaut nicht zu sehr anfärben und vor allem sollen sie in toxikologischer und dermatologischer Hinsicht unbedenklich sein.

Als Entwicklersubstanzen werden üblicherweise primäre aromatische Amine mit einer weitern in Para- oder Orthoposition befindlichen freien oder substituierten Hydroxy- oder Aminogruppe, ferner Diaminopyridinderivate, heterocyclische Hydrazonderivate und 4-Aminopyrazolonderivate eingesetzt. Als sogenannte Kupplersubstanzen werden m-Phenylendiaminderivate, Naphthole, Resorcinderivate und Pyrazolone verwendet.

Eine besondere Bedeutung kommt der Intensität der bei der oxidativen Kupplung gebildeten Farbnuancen und den Echtheitseigenschaften, insbesondere der Lichtechtheit zu. Aus DE-OS 35 43 345 waren bereits 4-Amino-2-aminomethylphenole als Oxidationshaarfarbstoffvorprodukte in Haarfärbemitteln bekannt. Es wurde nun gefunden, daß noch intensivere und beständigere Haaranfärbungen erreicht werden, wenn als Entwicklerkomponente 2-(Alkoxyalkylaminomethyl)-4-aminophenole der im folgenden näher beschriebenen Struktur eingesetzt werden.

Gegenstand der Erfindung sind 2-(Alkoxyalkylaminomethyl)-4-aminophenole der Formel (I)

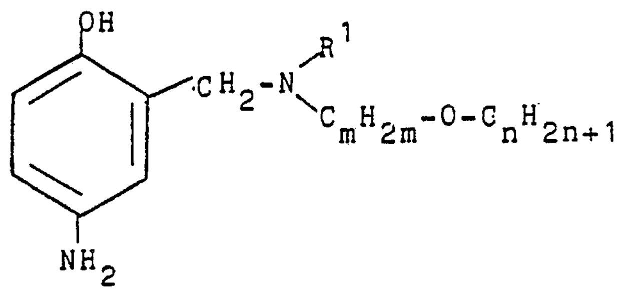

(I)

in der $R^1$ Wasserstoff, eine Alkylgruppe mit 1 - 4 C-Atomen, eine Hydroxyalkylgruppe mit 2 - 4 C-Atomen, eine Aminoalkylgruppe mit 2 - 4 C-Atomen, die an Stickstoffatom durch eine oder zwei Alkylgruppen mit 1 - 4 C-Atomen substituiert sein kann oder deren Stickstoffatom einem Piperidin-, Morpholin-, Pyrrolidin- oder Piperazin-Ring angehört oder eine Gruppe $-C_mH_{2m}-O-C_nH_{2n+1}$ ist, und worin m eine ganze Zahl von 2 - 4 und n eine ganze Zahl von 1 - 4 ist und deren wasserlösliche Salze. Von den Verbindungen der Formel (I) kommt solchen, in denen $R^1$ Wasserstoff ist, die größte Bedeutung zu.

Die Verbindungen der Formel (I) sind neu. Sie lassen sich aber auf an sich üblichem Wege dadurch herstellen, daß man 2-Chlormethyl-4-nitrophenol mit einem Amin der Formel (II)

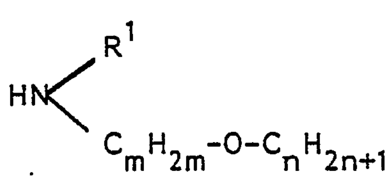

(II)

in der $R^1$, m und n die gleiche Bedeutung wie in Formel (I) haben, zur Umsetzung bringt und in dem dabei entstehenden 2-(Alkoxyalkylaminomethyl)-4-nitrophenol die Nitrogruppe katalytisch zur Aminogruppe hydriert.

Die neuen Verbindungen der Formel (I) oder deren Salze eignen sich zur Verwendung als Oxidationsfarbstoffvorprodukte vom Entwicklertyp in Haarfärbemitteln. Sie vermögen unter der Einwirkung von Oxidationsmitteln Farbstoffe auszubilden. Besonders intensive und brillante Farbstoffe werden jedoch durch oxidative Kupplung in Gegenwart von Kupplersubstanzen gebildet.

Ein weiterer Erfindungsgegenstand sind Haarfärbemittel mit einem Gehalt an Oxidationsfarbstoffvorprodukten in einem kosmetischen Träger, die als Oxidationsfarbstoffvorprodukte 2-(Alkoxyalkylaminomethyl)-4-aminophenole der Formel (I) oder deren wasserlösliche Salze als Enticklerkomponente neben üblichen Entwickler-oder Kupplerkomponenten und gegebenenfalls direktziehenden Haarfarbstoffen enthalten. Unter den zahlreichen bekannten Kupplersubstanzen sind für die neuen Entwickler der Formel (I) besonders solche mit phenolischen Hydroxylgruppen und solche vom Typ der Bis-(2,4-diaminophenyl)-alkane und der Bis-(2,4-diaminophenoxy)-alkane geeignet, wie sie in DE-OS 32 35 615 und DE-OS 28 52 272 beschrieben sind. Eine weitere, besonders bevorzugte Ausführungsform der Erfindung sind daher Haarfärbemittel der obengenannten Art, die als Kupplerkomponente Phenole, Naphthole, Resorcine und/oder 1,3-Bis-(2,4-diaminophenyl)-alkane oder 1,3-Bis-(2,4-diaminophenoxy)-alkane enthalten. Solche Haarfärbemittel liefern besonders intensive Färbungen im Bereich rotbrauner bis olivbrauner Nuancen.

Die erfindungsgemäßen 2-(Alkoxyalkylaminomethyl)-4-aminophenole der Formel (I) können entweder als solche oder in Form ihrer wasserlöslichen Salze mit anorganischen oder organischen Säuren, z.B. als Hydrochloride, Sulfate, Phosphate, Acetate, Propionate, Lactate oder Citrate isoliert und in Haarfärbemitteln eingesetzt werden.

In den erfindungsgemäßen Haarfärbemitteln werden die Entwicklersubstanzen und die Kupplersubstanzen im allgemeinen in äquimolaren Mengen eingesetzt, ein gewisser Überschluß einzelner Oxidationsfarbstoffvorprodukte ist jedoch nicht nachteilig, so daß Entwicklersubstanzen und Kupplersubstanzen in einem Molverhältnis von 1 : 0,5 bis 1 : 2 eingesetzt werden können. Die erfindungsgemäßen 2-Alkoxyalkylaminomethyl)-4-aminophenole der Formel (I) oder deren Salze können in einer Menge von 0,05 bis 10 Millimol pro 100 g des Haarfärbemittels verwendet werden. Es ist dabei nicht erforderlich, daß die Verbindungen der Formel (I) einheitliche Verbindungen sind. Vielmehr können auch Gemische der Verbindungen zum Einsatz kommen.

Zur Modifikation der Haaranfärbung können den erfindungsgemäßen Haarfärbemitteln auch bekannte direktziehende Haarfarbstoffe, z.B. Nitrophenylendiaminderivate, Anthrachinonfarbstoffe oder Indophenole zugesetzt werden.

Zur Herstellung der erfindungsgemäßen Haarfärbemittel werden die Oxidationsfarbstoffvorprodukte und gegebenenfalls direktziehende Farbstoffe in einen geeigneten kosmetischen Träger eingearbeitet. Solche Träger sind z.B. Cremes, Emulsionen, Gele oder auch tensidhaltige, schäumende Lösungen, z.B. Shampoos oder andere Zubereitungen, die für die Anwendung auf dem Haar geeignet sind. Übliche Bestandteile solcher kosmetischer Zubereitungen sind z.B. Netz- und Emulgiermittel wie anionische, nichtionische oder ampholytische Tenside, z.B. Fettalkoholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel, wie z.B. Methyl- oder Hydroxyethylcellulose, Stärke, Fettalkohole, Paraffinöle, Fettsäuren, ferner Parfümöle und haarpflegende Zusätze, wie z.B. wasserlösliche kationische Polymere, Proteinderivate, Pantothensäure und Cholesterin.

Die Bestandteile der kosmetischen Träger werden zur Herstellung der erfindungsgemäßen Haarfärbemittel in für diese Zwecke üblichen Mengen eingesetzt, z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt. Die Oxidationsfarbstoffvorprodukte werden in Mengen von 0,2 bis 5 Gew.-%, vorzugsweise 1 bis 3 Gew.-% des gesamten Färbemittels in den Träger gemischt.

Eine bevorzugte Anwendungsform für die erfindungsgemäßen 2-(Alkoxyalkylaminomethyl)-4-aminophenole der Formel (I) sind Cremehaarfärbemittel in Form einer Öl-in-Wasser-Emulsion mit einem Gehalt von

1 bis 10 Millimol pro 100 g an Entwicklerkomponenten,

1 bis 10 Millimol pro 100 g an Kupplerkomponenten,

1 bis 10 Gew.-% eines Alkyl($C_{10}$-$C_{18}$)-sulfat- oder Alkyl($C_{10}$-$C_{16}$)-ethersulfattensids,

5 bis 20 Gew-% eines Fettalkoholgemisches mit 12 bis 18 C-Atomen,

0,1 bis 2 Gew.-% eines Oxisationsinhibitors, bevorzugt aus der Gruppe Alkalisulfit, Alkali-ascorbat oder Alkali-dithionit

sowie Ammoniak in einer Menge, um den pH-Wert der Emulsion auf einen Wert zwischen 8 und 10 einzustellen.

Das genannte Alkylsulfat- oder Alkylethersulfattensid kann als Alkali-, Ammonium- oder Alkanolammoniumsalz mit 2 oder 3 C-Atomen in der Alkanolgruppe vorliegen, z.B. als Natrium-, Triethanolammonium- oder Isopropanolammoniumsalz. Als Alkyl-$C_{10}$-$C_{16}$-ethersulfattensid kann ein Schwefelsäuremonoestersalz eines Anlagerungsproduktes von 1 bis 10 Mol Ethylenoxid an einen $C_{10}$-$C_{16}$-Fettalkohol eingesetzt werden.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxodisulfat in Betracht.

Die Anwendung der erfindungsgemäßen Haarfärbemittel kann, unabhängig von der Art der kosmetischen Zubereitung, z.B. als Creme, Gel oder Shampoo, im schwach sauren, neutralen oder alkalischen Milieu erfolgen. Bevorzugt ist die Anwendung der Haarfärbemittel in einem pH-Bereich von 8 bis 10. Die Anwendungstemperaturen können in einem Bereich zwischen 15 °C und 40 °C liegen.

Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z.B. eine Färbeshampoo verwendet wurde.

Die nachfolgenden Beispiele sollen den Erfindungsgegenstand näher erläutern, ohne ihn jedoch hierauf zu beschränken.

## BEISPIELE

### 1. Herstellungsbeispiele

#### 1.1. N-(2-Hydroxy-5-aminobenzyl)-2-methoxyethylamin-dihydrochlorid

Stufe 1

Eine Mischung bestehend aus 18,8 g (0,1 Mol) 2-Chlormethyl-4-nitrophenol und 65,0 g (0,87 Mol) 2-Methoxyethylamin wurde für 3 Std. unter Rückfluß gekocht. Nach dem Einengen zur Trockene wurde mit Wasser aufgeschlemmt und abgesaugt. Nach dem Nachwaschen mit Wasser wurde bei 70 °C im Vakuum getrocknet.
Ausbeute: 19,4 g (68,7 % d.Th.);
Schmelzpunkt 160 - 162 °C.

Stufe 2

9,1 g (0,04 Mol) der Substanz nach Stufe 1 wurden in 300 ml Ethanol katalytisch reduziert (Pd/Kohle). Nach beendeter $H_2$-Aufnahme wurde vom Katalysator abfiltriert und die Lösung mit verdünnter Salzsäure angesäuert. Nach dem Einengen wurden 7,3 g (85 % d.Th.) erhalten.
Gelbe Kristalle; Schmelzpunkt 120 °C (Zers.).

#### 1.2. N-(2-Hydroxy-5-aminobenzyl)-1-methoxy-isopropylamin-dihydrochlorid

Stufe 1

Entsprechend Beispiel 1.1, Stufe 1 wurde eine Mischung aus 2-Chlor-methyl-4-nitrophenol 94 g (0,05 Mol) und 2-Amino-1-methoxypropan 35,7 g (0,4 Mol) 3 Std. auf 130 °C erhitzt.
Ausbeute: 9,5 g (78,6 % d.Th.) gelbes Pulver,
Schmelzpunkt: 188 - 195 °C

Stufe 2

Entsprechend Beispiel 1.1, Stufe 2 wurden 9,0 g (0,038 Mol in Stufe 1 erhaltenen Substanz in Ethanol katalytisch reduziert.
Gelbe Kristalle; Schmelzpunkt 175 - 181 °C;
Ausbeute: 8,6 g (81 % d.Th.)

1.3. N-(2-Hydroxy-5-aminobenzyl)-3-ethoxypropylamin-dihydrochlorid

Stufe 1

Analog zu Beispiel 1.1, Stufe 1, ausgehend von 41,3 g (0,4 Mol) 3-Ethoxypropylamin und 9,4 g (0,05 Mol) 2-Chlormethyl-4-nitrophenol.
Ausbeute: 8,0 g (63 % d.Th.); gelbe Kristalle,
Schmelzpunkte: 175 - 181 °C

Stufe 2

Analog Beispiel 1.1, Stufe 2 wurden 7,6 g (0,03 Mol) in Stufe 1 erhaltenen Substanz in Ethanol katalytisch reduziert.
Ausbeute: 6,1 g (68,5 % d.Th.); gelbe Kristalle,
Schmelzpunkt: 60 73 °C

1.4. N-(2-Hydroxy-5-aminobenzyl)-3-isopropoxypropylamin-dihydrochlorid

Stufe 1

Analog Beispiel 1.1, Stufe 1, ausgehend von 46,99 g (0,4 Mol) 3-Isopropoxypropylamin und 9,4 g (0,05 Mol) 2-Chlormethyl-4-nitrophenol.
Ausbeute: 7,5 g (56 % d.Th.); gelbe Kristalle,
Schmelzpunkt: 180 - 187 °C

Stufe 2

Analog Beispiel 1.1, Stufe 2 wurden 7,3 g in Stufe 1 erhaltenen Substanz in Ethanol katalytisch reduziert.
Ausbeute: 6,7 g (80 % d.Th.); beige Kristalle,
Schmelzpunkt: 75 - 85 °C

2. Anwendungsbeispiele

Es wurden erfindungsgemäße Haarfärbemittel in Form einer Haarfärbe-Cremeemulsion der folgenden Zusammensetzung hergestellt:

| | |
|---|---|
| Fettalkohol $C_{12-14}$ | 10,0 g |
| Fettalkohol $C_{12-14}$ + 2 EO-sulfat, Na-Salz, 28 %ig | 25,0 g |
| Wasser | 60,0 g |
| Entwickler gemäß Beispiel 1.1-1.4 | 7,5 mMol |
| Kupplerkomponente | 7,5 mMol |
| $Na_2SO_3$ (Inhibitor) | 1,0 g |
| konzentrierte Ammoniak-Lösung | bis pH = 9,5 |
| Wasser | ad 100 g |

Die Bestandteile wurden der Reihe nach miteinander vermischt. Nach Zugabe der Oxidationsfärbemittelvorprodukte und des Inhibitors wurde zunächst mit konzentrierter Ammoniak-Lösung der pH-Wert der Emulsion auf 9,5 eingestellt, dann wurde mit Wasser auf 100 g aufgefüllt.

Die oxidative Entwicklung der Färbung wurde mit 3 %iger Wasserstoffperoxidlösung als Oxidationsmittel durchgeführt. Hierzu wurden 100 g der Emulsion mit 50 g Wasserstoffperoxidlösung (3 %ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Als Kuppler wurden die folgenden Verbindungen eingesetzt:

K1 Resorcin
K2 m-Aminophenol
K3 5-Amino-2-methylphenol
K4 5-Amino-4-chlor-2-methylphenol
K5 5-Amino-6-chlor-2-methylphenol
K6 1,3-Bis-(2,4-diaminophenoxy)-propan
K7 1-Naphthol
K8 1,5-Dihydroxy-naphthalin
K9 1-Phenyl-3-amino-pyrazolon-5

Die Ergebnisse der Färbeversuche sind der Tabelle I zu entnehmen.

TABELLE I

| Beispiel Nr. | Entwickler gemäß | Kuppler | Nuance des gefärbten Haares |
|---|---|---|---|
| 2.1 | Beispiel 1.1 | K1 | olivbraun |
| 2.2 | 1.1 | K3 | madeirabraun |
| 2.3 | 1.1 | K4 | rotbraun |
| 2.4 | 1.1 | K5 | rotbraun |
| 2.5 | 1.1 | K6 | dunkelbraun |
| 2.6 | 1.1 | K9 | violettbraun |
| 2.7 | 1.2 | K1 | olivbraun |
| 2.8 | 1.2 | K2 | dunkelbraun |
| 2.9 | 1.2 | K3 | madeirabraun |
| 2.10 | 1.2 | K4 | rotbraun |
| 2.11 | 1.2 | K5 | rotbraun |
| 2.12 | 1.2 | K6 | dunkelrubin |
| 2.13 | 1.2 | K7 | graurubin |
| 2.14 | 1.2 | K8 | rotbraun |
| 2.15 | 1.2 | K9 | violettbraun |
| 2.16 | 1.3 | K1 | olivbraun |
| 2.17 | 1.3 | K3 | tizianrot |
| 2.18 | 1.3 | K6 | dunkelbraun |
| 2.19 | 1.4 | K1 | olivbraun |
| 2.20 | 1.4 | K3 | madeirabraun |
| 2.21 | 1.4 | K6 | dunkelbraun |

**Ansprüche**

1. 2-(Alkoxyalkyl-aminomethyl)-4-aminophenole der Formel I

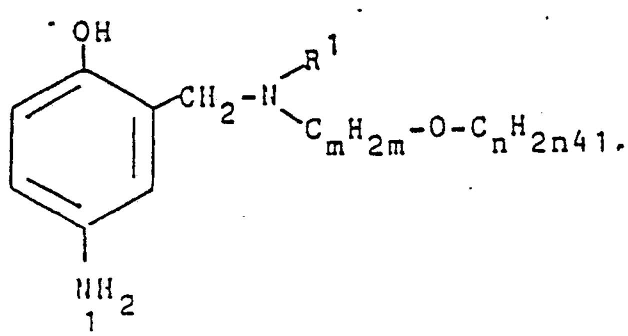

(I)

in der $R^1$ Wasserstoff, eine Alkylgruppe mit 1 - 4 C-Atomen, eine Hydroxyalkylgruppe mit 2 - 4 C-Atomen, eine Aminoalkylgruppe mit 2 - 4 C-Atomen, die am Stickstoffatom durch eine oder zwei Alkylgruppen mit 1 - 4 C-Atomen substituiert sein kann oder deren Stickstoffatom einem Piperidin-, Morpholin-, Pyrrolidin- oder Piperazin-Ring angehört oder einer Gruppe $-C_mH_{2m}-O-C_nH_{2n+1}$ ist und worin m eine ganze Zahl von 2 - 4 und n eine ganze Zahl von 1 - 4 ist und deren wasserlösliche Salze.

2. 2-(Alkoxyalkyl-aminomethyl)-4-aminophenole der Formel I gemäß Anspruch 1, dadurch gekennzeichnet, daß $R^1$ = Wasserstoff ist.

3. Verwendung der 2-(Alkoxyalkyl-aminoalkyl)-4-aminophenole der Formel I, gemäß Anspruch 1 oder 2 oder deren Salze als Oxidationsfarbstoffvorprodukte vom Entwicklertyp in Haarfärbemitteln.

4. Haarfärbemittel, enthaltend Oxidationsfarbstoffvorprodukte in einem kosmetischen Träger, dadurch gekennzeichnet, daß als Oxidationsfarbstoffvorprodukte 2-(Alkoxyalkyl-aminomethyl)-4-aminophenole der Formel I gemäß Anspruch 1 oder 2 oder deren wasserlösliche Salze als Entwicklerkomponente neben

üblichen Entwickler- und Kupplerkomponenten und ggf. direktziehenden Haarfarbstoffen enthalten sind.

5. Haarfärbemittel nach Anspruch 4, dadurch gekennzeichnet, daß als Kupplerkomponenten Phenole, Naphthole, Resorcine und/oder 1,3-Bis- (2,4-diaminophenyl)-alkane oder 1,3-Bis-(2,4-diaminophenoxy)-alkane enthalten sind.

6. Haarfärbemittel nach Anspruch 4 oder 5, dadurch gekennzeichnet, daß Entwicklersubstanzen und Kupplersubstanzen im Molverhältnis 1 : 0,5 bis 1 : 2 enthalten sind, daß der Gehalt an Oxidationsfarbstoffvorprodukten 0,2 bis 0,5 Gew.-% des Haarfärbemittels und der Gehalt an 2-(Alkoxyalkyl-aminomethyl)-4-aminophenol der Formel I, gemäß Patentanspruch 1 oder deren Salze 0,05 bis 10 Millimol pro 100 g des Haarfärbemittels beträgt.

Europäisches Patentamt

# EUROPÄISCHER RECHERCHENBERICHT

Nummer der Anmeldung

EP 89 11 6863

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 226 072 (WELLA)<br>* Ansprüche; Seite 11, Verbindung IX *<br>----- | 1-6 | C 07 C 217/10<br>C 07 D 245/12<br>A 61 K 7/13 |
| | | | **RECHERCHIERTE SACHGEBIETE (Int. Cl.5)** |
| | | | C 07 C 217/00 |

Der vorliegende Recherchenbericht wurde für alle Patentansprüche erstellt

| Recherchenort | Abschlußdatum der Recherche | Prüfer |
|---|---|---|
| DEN HAAG | 12-12-1989 | SANCHEZ Y GARCIA J.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)